(19) Europäisches Patentamt
European Patent Office
Office européen des brevets

(11) Publication number: **0 145 314**
**A1**

# (12) EUROPEAN PATENT APPLICATION

(21) Application number: 84307858.5

(22) Date of filing: 13.11.84

(51) Int. Cl.⁴: C 07 D 249/08
A 61 K 31/41, A 01 N 43/653

(30) Priority: 08.12.83 GB 8332788

(43) Date of publication of application: 19.06.85 Bulletin 85/25

(84) Designated Contracting States: AT BE CH DE FR GB IT LI LU NL SE

(71) Applicant: IMPERIAL CHEMICAL INDUSTRIES PLC
Imperial Chemical House Millbank
London SW1P 3JF(GB)

(72) Inventor: Boyle, Francis Thomas
Hinstock Mount Astbury Lane Ends
Congleton Cheshire(GB)

(74) Representative: Atkinson, John David et al,
Imperial Chemical Industries PLC Legal Department :
Patents PO Box 6 Bessemer Road
Welwyn garden City Herts, AL7 1HD(GB)

## (54) Antifungal triazole compound.

(57) The antifungal triazole compound 2-(2-fluoro-4-trifluoromethylphenyl)-1,3-di(1,2,4-triazol-1-yl)-2-propanol, processes for its manufacture, pharmaceutical, veterinary and plant antifungal compositions containing it, and its use in a method of combatting plant fungal diseases.

EP 0 145 314 A1

Croydon Printing Company Ltd

TITLE : ANTIFUNGAL TRIAZOLE COMPOUND

This invention relates to a novel antifungal triazole compound which is useful for the treatment of candidosis and human dermatophyte infections, to processes for its manufacture, to pharmaceutical, veterinary, plant antifungal and plant growth regulating compositions containing it, and to processes for controlling fungal infections of plants and processes for regulating plant growth.

Our European Patent Specification Number 44605 discloses a broad class of antifungal azole compounds of the formula I wherein $R^1$ is alkyl, cycloalkyl, aryl or aralkyl any of which may be optionally substituted, and $Y^1$ and $Y^2$ are =CH- or =N-, and their acid addition salts, metal complexes, ethers and esters.

United Kingdom Patent Applications Numbers 811739, 8131370 and 8206329, from which European Patent Application Number 82302075.5 claims priority, similarly disclose a broad class of triazole compounds of the formula II wherein R is naphthyl, biphenylyl or optionally-substituted phenyl, and their pharmaceutically acceptable salts.

Although the specific exemplified compounds disclosed in our European Patent Application Number 44605 are very effective antifungals and have relatively low gross toxicity, as measured by their $LD_{50}$ in mice, it is now known that the preferred exemplified compounds therein can cause teratological effects in the foetuses of pregnant female rats at doses which are below the maternally toxic dose.

It is an object of the present invention to provide a compound of the formula I or II which is not teratogenic in either the rat or the rabbit at doses which are lower than the maternally-toxic dose.

Thus according to the invention there is provided 2-(2-fluoro-4-trifluoromethylphenyl)-1,3-di(1,2,4-triazol-1-yl)-2-propanol of the general formula II, in which R is 2-fluoro-4-trifluoromethylphenyl, and the acid addition salts thereof.

Suitable acid addition salts are for example, the hydrochloride, hydrobromide, nitrate, sulphate and toluene-p-sulphonate.

The compound of the invention may be manufactured by methods generally known per se for the manufacture of chemically analogous compounds. Thus, the following processes are provided as further features of this invention:-

a) the reaction of 2-(2-fluoro-4-trifluoromethylphenyl)-2-(1,2,4-triazol-1-ylmethyl)oxirane with 1,2,4-triazole in the presence of a strong base or with preformed 1,2,4-triazole sodium in an inert solvent;

b) the reaction of a compound of the formula III or IV, wherein X is an easily displaceable leaving group, for example a halogen such as chlorine, bromine or iodine or a sulphonyl group such as tosyl or mesyl, with 1,2,4-triazole in the presence of a strong base, or with preformed 1,2,4-triazole sodium in an inert solvent.

c) the reaction of an epoxide of the formula V, wherein X has the meaning stated above, with 1,2,4-triazole in the presence of a strong base, or with preformed 1,2,4-triazole sodium in an inert solvent.

d) the reaction of 1,3-di(1,2,4-triazol-1-yl)-acetone with a 2-fluoro-4-trifluoromethylphenyl

Grignard reagent or with 2-fluoro-4-trifluoromethyl-phenyl-lithium; or

e) the reaction of 2-fluoro-α-(1,2,4-triazol-1-yl)-4-(trifluoromethyl)acetophenone either with a triphenyl(1,2,4-triazol-1-ylmethyl)phosphonium halide, for example the bromide or iodide, or with a di(lower alkyl) (1,2,4-triazol-1-yl)phosphonate.

In processes a), b) and c) a suitable strong base is, for example, an alkali metal hydride, hydroxide or 1-6C alkoxide, for example sodium hydride, sodium or potassium hydroxide, sodium methoxide or ethoxide or potassium tert-butoxide, in an inert solvent. When preformed 1,2,4-triazole sodium is used, a suitable inert solvent is, for example, dimethylformamide or butanol.

In process e), a suitable lower alkyl radical is, for example, such a radical of 1 to 6 carbon atoms, for example a methyl or ethyl radical.

2-(2-Fluoro-4-trifluromethylphenyl)-2-(1,2,4-triazol-1-ylmethyl)oxirane, which is used as the starting material in process a) above, may be manufactured by, for example, brominating 2-fluoro-4-trifluoromethylacetophenone, reacting the 2-fluoro-4-trifluoromethylphenacyl bromide thus obtained with 1,2,4-triazole in the presence of a strong base, and then reacting the 2-fluoro-α-(1,2,4-triazol-1-yl)-4-trifluoromethylacetophenone thus obtained with dimethylsulphonium methylide or with dimethyloxosulphonium methylide to obtain the required oxirane starting material.

The compound of the formula III, used as the starting material in process b) above, may be manufactured by, for example, reacting α-(1,2,4-

triazol-1-yl)acetophenone with a methyl Grignard reagent to form 1-(2-fluoro-4-trifluoromethylphenyl)-2-(1,2,4-triazol-1-yl)2-propanol, which is then dehydrated, for example with toluene-p-sulphonic acid in toluene, and the resulting olefin, 2-fluoro- α -(1,2,4-triazol-1-yl)-4-trifluoromethylstyrene, is treated with, say, bromine in acetic acid to produce the required starting material of the formula III, X = Br.

The compound of the formula IV, which may be used as a starting material in process b), may be manufactured by, for example, reacting a ketone of the formula $XCH_2COCH_2X$, wherein X has the meaning stated above, with a 2-fluoro-4-trifluoromethylphenyl Grignard reagent, or 2-fluoro-4-trifluoromethylphenyl-lithium.

The epoxide of the formula V, used as the starting material in process c) above, may be manufactured by, for example, reacting a phenacyl compound of the formula $RCOCH_2X$, wherein X has the meaning stated above, with trimethyloxosulphonium iodide and a strong base.

Alternatively, the epoxide V may be manufactured by thermolysis of a compound of the formula IV.

1,3-Di(1,2,4-triazol-1-yl)acetone, which is used as starting material in process d) above, may be manufactured by, for example, reacting 1,3-dichloroacetone with 1,2,4-triazole.

The phosphonium and phosphonate reagents used as starting materials in process e) above, may be manufactured by reacting 1-chloromethyl-1,2,4-triazole with either triphenylphosphine, as described in European Patent Publication No.60222, or with potassium diethyl phosphite.

As indicated above, the compounds of the invention possess antifungal properties which make them useful in the treatment of candidosis and human dermatophyte infections.

This antifungal activity against Candida albicans, a causative fungus of candidosis, and Trichophyton mentagrophytes, var. quinkeanum, a causative fungus of ringworm, was demonstrated as follows:-

Female mice of around 30 g. weight are injected sub-cutaneously on a Friday with 0.5 mg. of oestradiol benzoate. The following Monday (day 0) they are clipped on the back and then dosed orally with the test compound. They are then inoculated with Candida albicans in the vagina and Trichophyton mentagrophytes var. quinkeanum on the back, and then given a second dose of the same compound. Dosing is repeated once daily on days 1-4. On day 7 skin lesions are scored visually and vaginal samples taken for culture on agar. Groups of 5 mice are used and the compound is dosed initially at a level of 250 mg./kg. The dose is then reduced sequentially until a minimum effective dose (MED) is found. The MED for 2-(2-fluoro-4-trifluoromethylphenyl)-1,3-di(1,2,4-triazol-1-yl)-2-propanol in this test was 5 mg. per kg.

As indicated above, the compound of this invention has been found not to be teratogenic at doses up to the maternally toxic dose. In both the rabbit and the rat, the maternally toxic dose was taken to be the lowest dose at which impaired maternal weight gain was observed. In the rabbit, this dose was 10 mg./kg. and in the rat between 25 and 50 mg./kg. In neither species was any teratological effect seen in the foetuses at these doses.

Thus, according to a further feature of the invention there is provided a pharmaceutical or veterinary antifungal composition which comprises 2-(2-fluoro-4-trifluoromethylphenyl)-1,3-di(1,2,4-triazol-1-yl)-2-propanol together with a pharmaceutically or veterinarily acceptable diluent or carrier.

The composition of the invention may be in a conventional pharmaceutical form suitable for oral administration, for example a tablet, a capsule, an emulsion or an aqueous or oily solution or suspension, or suitable for topical application, for example a cream, ointment or gel. The composition may contain conventional pharmaceutical excipients, and may be manufactured by conventional pharmaceutical techniques.

Preferred pharmaceutical or veterinary compositions of the invention are compositions suitable for oral administration, and particularly tablets and capsules.

In man, the daily intake of the compound for the treatment of fungal diseases, for example candidosis or human dermatophyte infections, is from 5 to 250mg., preferably 25 to 100mg., and for this purpose, tablets or capsules containing between 5 and 25mg. of the compound are an appropriate dosage form.

The compound of the invention also possesses antifungal properties which are useful in combatting a wide variety of plant fungal diseases.

The compound can move acropetally when applied to the plant tissue, and can also be volatile enough to be active in the vapour phase against fungi on the plant.

The compound may be used as such for plant fungicidal purposes but is more conveniently formulated into compositions for such usage. The invention thus provides also a plant antifungal composition comprising 2-(2-fluoro-4-trifluoromethylphenyl)-1,3-di(1,2,4-triazol-1-yl)-2-propanol and a non-pharmaceutical carrier or diluent.

The invention also provides a method of combatting fungal diseases in a plant, which method comprising applying to the plant, to seed of the plant or to the locus of the plant or seed, an antifungally effective amount of 2-(2-fluoro-4-trifluoromethylphenyl)-1,3-di(1,2,4-triazol-1-yl)-2-propanol.

The compound can be applied in a number of ways, for example it can be applied, formulated or unformulated, directly to the foliage of a plant, to seeds or to the medium in which plants are growing or are to be planted, or it can be sprayed on, dusted on or applied as a cream or paste formulation, or it can be applied as a vapour. Application can be to any part of the plant, bush or tree, for example to the foliage, stems, branches or roots, or to soil surrounding the roots, or to the seed before it is planted.

The term "plant" as used herein includes seedlings, bushes and trees. Furthermore, the fungicidal method of the invention includes preventative, protectant, prophylactic and eradicant treatment.

The compound is preferably used for agricultural and horticultural purposes in the form of a composition. The type of composition used in any instance will depend upon the particular purpose envisaged, and the choice of a suitable conventional composition, and the method by which such a composition may be manufactured, are apparent to those skilled in the art.

The invention is illustrated, but not limited, by the following Examples, in which temperatures are given in degrees Celsius.

Example 1

1,3-Dichloro-2-(2-fluoro-4-trifluoromethylphenyl)-2-propanol (15 g.) and 1,2,4-triazole (7.5 g.) were added to a solution of sodium hydride (3.3 g. of 50% dispersion in oil) in tert-butyl alcohol (100 ml.) and heated at 100° for 16 hours. The reaction mixture was evaporated to dryness and the residue was partitioned between ethyl acetate and water. The organic layer was separated, washed with water, dried with anhydrous magnesium sulphate and filtered, and the filtrate was evaporated to dryness. The residual gum was crystallised from a mixture of ethyl acetate and petroleum ether (b.p. 60-80°C.) to give a cream-coloured solid. The solid was subjected to medium pressure liquid chromatography on silica (grade K60) using chloroform and methanol/chloroform 10/90 v/v as eluting solvents, and the product was recrystallised from a mixture of ethyl acetate and petroleum ether (b.p. 60-80°C.) to give 2-(2-fluoro-4-trifluoromethylphenyl)-1,3-di(1,2,4-triazol-1-yl)-2-propanol m.p. 176-8°C.

The 1,3-dichloro-2-(2-fluoro-4-trifluoromethylphenyl)-2-propanol used as starting material may be prepared as follows:-

2-Bromo-5-(trifluoromethyl)aniline (50.1 g.) was added to concentrated hydrochloric acid (126 ml.) and stirred at 0°C. A solution of sodium nitrite (16.1 g.) in water (21 ml.) was added dropwise, keeping the temperature between 0° and 5°C. The mixture was stirred at 0°C. for 2 hours, then a solution of sodium fluoroborate (46 g.) in water (55 ml.) was added dropwise. The precipitate was filtered and washed with 5% w/v aqueous sodium fluoroborate (80 ml.), followed by ice-cooled methanol (25 ml.), then diethyl ether

(3 x 150 ml.). The resulting solid was dried over phosphorus pentoxide overnight, then decomposed in 20 g. batches by heating at 200-210°C. The product was extracted from the residue by extracting with diethyl ether (3 x 100 ml.), washing with aqueous sodium bicarbonate solution, drying with anhydrous magnesium sulphate and evaporating the filtered solution to dryness to give a brown oil, which was distilled at 15 mm. of mercury pressure to give 1-bromo-2-fluoro-4-trifluoromethylbenzene as a colourless liquid b.p. 56°C.

1-Bromo-2-fluoro-4-trifluoromethylbenzene (7.2 g.) was dissolved in diethyl ether (50 ml.) and cooled to -70°C. under an atmosphere of argon. N-Butyl lithium (22 ml., 1.6 M in hexane) was added dropwise and the mixture was stirred at -70°C. for 30 minutes. A solution of 1,3-dichloroacetone (4 g.) in diethyl ether (25 ml.) was added dropwise at -70°C. and the mixture was stirred at -70°C. for 1 hour. A solution of glacial acetic acid (5 ml.) in diethyl ether (10 ml.) was added dropwise and the temperature was allowed to rise to room temperature. The reaction mixture was diluted with water and extracted twice with diethyl ether (100 ml.). The ether extracts were combined, dried with anhydrous magnesium sulphate and evaporated to dryness. Toluene (100 ml.) was added and the mixture was re-evaporated to dryness to give 1,3-dichloro-2-(2-fluoro-4-trifluoromethylphenyl)-2-propanol as a pale yellow oil.

Example 2

2-Chloromethyl-2-(2-fluoro-4-trifluoromethylphenyl)oxirane (4 g.) was dissolved in tert-butyl alcohol (20 ml.) and added to a mixture of potassium tert-butoxide (1.6 g.) and 1,2,4-triazole (2.2 g.) in

tert-butyl alcohol (20 ml.), and the mixture was heated at 100°C. for 16 hours. The solvent was evaporated and the residue was partitioned between ethyl acetate and water. The organic layer was separated, washed with water and dried with anhydrous mangesium sulphate, and the solution was evaporated to dryness. The residual gum was subjected to medium pressure liquid chromatography on silica (K60 grade) using chloroform as eluting solvent, and the product so obtained was recrystallised from a mixture of ethyl acetate and petroleum ether (b.p. 60-80°C.) to give 2-(2-fluoro-4-trifluoromethylphenyl)-1,3-di(1,2,4-triazol-1-yl)-2-propanol (1.7 g.) m.p. 176-8°C.

The 2-chloromethyl-2-(2-fluoro-4-trifluoromethylphenyl)oxirane used as starting material in the above example may be prepared as follows:-

1,3-Dichloro-2-(2-fluoro-4-trifluoromethylphenyl)-2-propanol (7 g.) was added to a solution of sodium hydride (1.45 g. of 48% dispersion in oil) in tert-butyl alcohol (30 ml.) and heated at 100°C. for 1 hour. The mixture was evaporated to dryness and the residue was partitioned between ethyl acetate and water. The organic layer was separated, washed with water and dried with anhydrous magnesium sulphate, and the filtered solution was evaporated to dryness to give 2-chloromethyl-2-(2-fluoro-4-trifluoromethylphenyl)-oxirane as a yellow oil.

Example 3

A mixture of 5,10,15,20 or 25g. of 2-(2-fluoro-4-trifluoromethylphenyl)-1,3-di(1,2,4-triazol-1-yl)-2-propanol with calcium carbonate (70g.) and 10% maize starch (200g.) was dried and then passed through a 16 mesh screen. (British Standard 410:1962) Magnesium stearate (5g.) was added, and the granules so obtained were compressed to give a range of tablets weighing 280

to 300mg. and containing respectively 5, 10, 15, 20 or 25mg. of the antifungal compound, suitable for oral administration for therapeutic purposes.

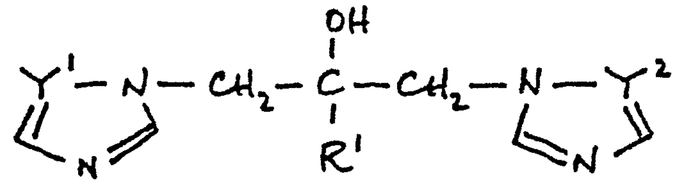

I

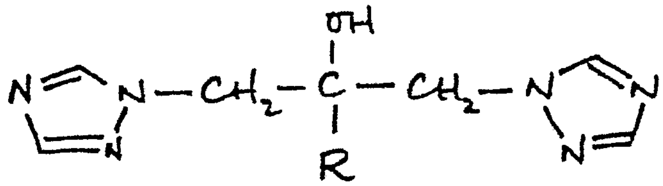

II

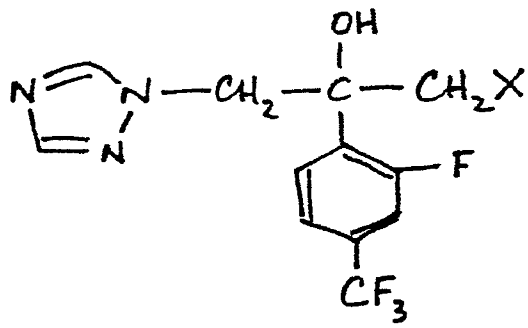

III

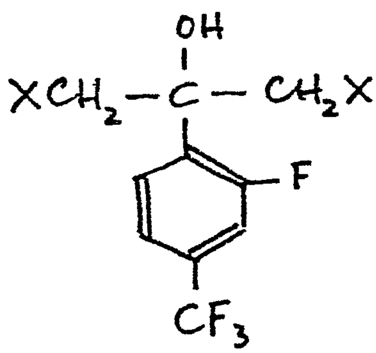

IV

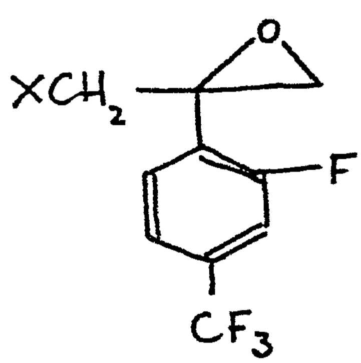

V

What we claim is:-

1. 2-(2-Fluoro-4-trifluoromethylphenyl)-1,3-di-(1,2,4-triazol-1-yl)-2-propanol, and the acid addition salts thereof.

2. A process for the manufacture of 2-(2-fluoro-4-trifluoromethylphenyl)-1,3-di(1,2,4-triazol-1-yl)-2-propanol which comprises

(a) the reaction of 2-(2-fluoro-4-trifluoromethyl-phenyl)-2-(1,2,4-triazol-1-ylmethyl)oxirane with 1,2,4-triazole in the presence of a strong base or with preformed 1,2,4-triazole sodium in an inert solvent;

(b) the reaction of a compound of the formula III or IV, wherein X is an easily displaceable leaving group, with 1,2,4-triazole in the presence of a strong base, or with preformed 1,2,4-triazole sodium in an inert solvent.

(c) the reaction of an epoxide of the formula V, wherein X has the meaning stated above, with 1,2,4-triazole in the presence of a strong base, or with preformed 1,2,4-triazole sodium in an inert solvent.

(d) the reaction of 1,3-di(1,2,4-triazol-1-yl)-acetone with a 2-fluoro-4-trifluoromethylphenyl Grignard reagent or with 2-fluoro-4-trifluoromethyl-phenyl-lithium; or

(e) the reaction of 2-fluoro-α-(1,2,4-triazol-1-yl)-4-(trifluoromethyl)acetophenone either with a triphenyl(1,2,4-triazol-1-ylmethyl)phosphonium halide, or with a di(lower alkyl) (1,2,4-triazole-1-yl)phosphonate.

3. A pharmaceutical or veterinary antifungal composition which comprises 2-(2-fluoro-4-trifluoromethylphenyl)-1,3-di(1,2,4-triazol-1-yl)-2-propanol together with a pharmaceutically or veterinarily acceptable diluent or carrier.

4. A method of combatting fungal diseases in a plant, which method comprises applying to the plant, to seed of the plant or the locus of the plant or seed an antifungally effective amount of 2-(2-fluoro-4-trifluoromethylphenyl)-1,3-di(1,2,4-triazol-1-yl)-2-propanol.

DS32959
SC24
16 Oct 84

Claim for Austria

A process for the manufacture of 2-(2-fluoro-4-trifluoromethylphenyl)-1,3-di(1,2,4-triazol-1-yl)-2-propanol which comprises

(a) the reaction of 2-(2-fluoro-4-trifluoromethylphenyl)-2-(1,2,4-triazol-1-ylmethyl)oxirane with 1,2,4-triazole in the presence of a strong base or with preformed 1,2,4-triazole sodium in an inert solvent;

(b) the reaction of a compound of the formula III or IV, wherein X is an easily displaceable leaving group, with 1,2,4-triazole in the presence of a strong base, or with preformed 1,2,4-triazole sodium in an inert solvent.

(c) the reaction of an epoxide of the formula V, wherein X has the meaning stated above, with 1,2,4-triazole in the presence of a strong base, or with preformed 1,2,4-triazole sodium in an inert solvent.

(d) the reaction of 1,3-di(1,2,4-triazol-1-yl)-acetone with a 2-fluoro-4-trifluoromethylphenyl Grignard reagent or with 2-fluoro-4-trifluoromethyl-phenyl-lithium; or

(e) the reaction of 2-fluoro- -(1,2,4-triazol-1-yl)-4-(trifluoromethyl)acetophenone either with a triphenyl(1,2,4-triazol-1-ylmethyl)phosphonium halide, or with a di(lower alkyl) (1,2,4-triazole-1-yl)phosphonate.

DS32050/EP Austria
SC24
16 Oct 84

European Patent Office

# EUROPEAN SEARCH REPORT

0145314

Application number

EP 84307858.5

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| P,A | EP - A2 - 0 096 569 (PFIZER) * Formula I; page 2, lines 13-15; abstract * | 1,3,4 | C 07 D 249/08<br>A 61 K 31/41<br>A 01 N 43/653 |
| | -- | | |
| A | EP - A3 - 0 052 424 (ICI) * Abstract * | 1 | |
| | ---- | | |

TECHNICAL FIELDS SEARCHED (Int Cl 4)

C 07 D 249/00

The present search report has been drawn up for all claims

| Place of search | Date of completion of the search | Examiner |
|---|---|---|
| VIENNA | 11-02-1985 | HAMMER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82